# EUROPEAN PATENT APPLICATION

(11) **EP 3 679 957 A1**
(43) Date of publication of application: **15.07.2020**
(21) Application number: 18853123.0
(22) Date of filing: 06.09.2018
(51) Int. Cl.: A61M 1/00, A61M 25/00, A61M 25/10, A61M 25/14

(54) **URINARY CATHETER**

(30) Priority: 07.09.2017 JP 2017172174
(71) Applicant: Otsuka Techno Corporation, Naruto-shi, Tokushima 771-0360 (JP)
(72) Inventor: INOUE, Shinichiro, Naruto-shi Tokushima 771-0360 (JP); MASUDA, Tetsuya, Naruto-shi Tokushima 771-0360 (JP); TSUKUI, Nobuo, Naruto-shi Tokushima 771-0360 (JP)
(74) Representative: Witte, Weller & Partner Patentanwälte mbB
(86) International application number: PCT/JP2018/033107
(87) International publication number: WO 2019/049955

(57) **Abstract**

Provided is a urinary catheter capable of easily draining urine stored from an inner wall of a bladder up to the top of a balloon portion, while the balloon portion maintains a function for preventing falling-off of the catheter. There is provided a urinary catheter 1 which includes a catheter body 2, a urine draining lumen 11 which is formed inside the catheter body 2, a balloon 4 which has a first end portion 25 fixed so as to surround the catheter body 2 at a tip end portion side of the catheter body 2 and a second end portion 26 and also has a linear recessed portion 38 extending from the first end portion 25 toward the second end portion 26 along a circumferential surface 31 thereof in an expanded state, a first urine draining port 9 which is formed in the catheter body 2 so as to be closer to the tip end portion side than the balloon 4 to communicate with the urine draining lumen 11, and a second urine draining port 37 which is formed in the vicinity of the first end portion 25 of the balloon 4 in the catheter body 2 to communicate with the urine draining lumen 11.

## Description

### Technical Field

The present invention relates to a urinary catheter.

### Background Art

A urinary catheter is conventionally known as a medical device for assisting urethral catheterization of a patient who has difficulty in urination.

For example, Patent Literature 1 discloses a urinary catheter having a rod-shaped catheter body, a balloon portion formed at one end of the catheter body and an operating portion formed at the other end of the catheter body.

The urinary catheter of Patent Literature 1 is used, for example, by performing the following processes (1) to (4) in this order.
(1) Disinfect an area around an external urethral opening of the penis with a swab soaked in disinfectant such as povidone-iodine.
(2) Open the packaging of the urinary catheter and apply a water-soluble lubricant to the urinary catheter.
(3) Carefully insert the urinary catheter from the external urethral opening. After a balloon portion has reached the inside of a bladder, a specified volume of sterile water is slowly infused into the balloon portion.
(4) Slightly pull the urinary catheter unit until the balloon portion comes into contact with the bladder neck and place the catheter there.

### Citation List

### Patent Literature

Patent Literature 1: Japanese Patent No.5318925

### Summary of Invention

### Technical Problem

In a conventional configuration, a balloon portion in contact with the bladder neck will serve as a function for preventing falling-off of a catheter. An opening portion which is used for draining urine from a bladder is formed so as to be closer to the tip end side of the catheter than the balloon portion. Therefore, it is difficult to drain urine stored, for example, from the bladder neck up to the top of the balloon portion.

An object of the present invention is to provide a urinary catheter capable of easily draining urine stored from an inner wall of the bladder up to the top of a balloon portion, while the balloon portion maintains a function for preventing falling-off of the catheter.

### Solution to Problem

A urinary catheter of the present invention includes a catheter body composed of a flexible tube having a tip end portion which is placed inside a bladder of a patient and a base portion on the opposite side thereof, a urine draining lumen which is formed inside the catheter body to lead urine inside the bladder of the patient to the outside of the body of the patient, a balloon portion which is provided with a first end portion fixed so as to surround the catheter body on the tip end portion side of the catheter body and a second end portion closer to the tip end portion side than the first end portion and which is composed of a membrane in which a portion between the first end portion and the second end portion expands spherically inside the bladder of the patient, and a balloon portion which has a linear recessed portion extending from the first end portion toward the second end portion along a circumferential surface thereof in an expanded state, a liquid introducing lumen which is formed by extending along the urine draining lumen inside the catheter body so as to communicate with the balloon portion, thereby introducing an expansion liquid into the balloon portion, a first urine draining port which is formed in the catheter body so as to be closer to the tip end portion side than the balloon portion to communicate with the urine draining lumen, and a second urine draining port which is formed in the vicinity of the first end portion of the balloon portion in the catheter body to communicate with the urine draining lumen.

In the urinary catheter of the present invention, the balloon portion may be such that a site where the recessed portion is formed is thicker in membrane thickness than a site other than that in a cross sectional view of the catheter body in a radial direction.

In the urinary catheter of the present invention, the balloon portion may be such that a site where the recessed portion is formed is 0.3 mm to 1.5 mm in membrane thickness and a site other than the site where the recessed portion is formed is 0.05 mm to 0.7 mm in membrane thickness.

In the urinary catheter of the present invention, the plurality of linear recessed portions may be formed at an interval from each other, in a cross sectional view of the catheter body in a radial direction.

In the urinary catheter of the present invention, an inner wall of the balloon portion may include a raised portion in which a site where the recessed portion has been formed selectively protrudes in a cross sectional view of the catheter body in an expanded state in a radial direction.

### Advantageous Effects of Invention

According to the urinary catheter of the present invention, in a state that the expanded balloon portion is in contact with the inner wall of a bladder, a certain space is formed between the balloon portion and the inner wall of the bladder. Then, the linear recessed portion is formed at the balloon portion so as to enter into this space. Thereby, even when a liquid surface of urine inside the bladder is at a position lower than the first urine draining port, urine present at a position lower than the first urine draining port is allowed to flow downward along the recessed portion, guided to the space and can be drained through the second urine draining port which is exposed to the space. As a result, it is possible to easily drain urine stored from the inner wall of the bladder up to the top of the balloon portion, while the balloon portion maintains a function for preventing falling-off of the catheter.

### Brief Description of Drawings

FIG. 1 is a drawing which shows a state that a urinary catheter according to a preferred embodiment of the present invention is used, and shows a state that the balloon has not yet expanded inside a bladder.
FIG. 2 is a drawing which shows a state that the urinary catheter according to a preferred embodiment of the present invention is used, and shows an expanded state of the balloon inside a bladder.
FIG. 3 is a plan view of the urinary catheter according to a preferred embodiment of the present invention.
FIG. 4 is a side view of the urinary catheter according to a preferred embodiment of the present invention.
FIG. 5 is a sectional view taken along section line V-V in FIG. 4.
FIG. 6 is an enlarged view of a portion surrounded by alternate long and two short dashed line VI in FIG. 5, and a drawing which shows a state that the balloon is not yet used.
FIG. 7 is an enlarged view of the portion surrounded by alternate long and two short dashed line VI in FIG. 5, and a drawing which shows a state that the balloon is used.
FIG. 8 is an enlarged view of a portion surrounded by alternate long and two short dashed line VIII in FIG. 5.
FIG. 9 is an enlarged view of a portion surrounded by alternate long and two short dashed line IX in FIG. 4.
FIG. 10 is a drawing which shows a positional relationship between the first urine draining port and the second urine draining port.
FIG. 11 is a sectional view taken along section line XI-XI in FIG. 3, and shows a state that the shape of a catheter body is not yet compressed.
FIG. 12 is a sectional view taken along section line XI-XI in FIG. 3, and shows a state that the shape of the catheter body is compressed.
FIG. 13 is a drawing which shows a modified example of the catheter body, and shows a state that the shape of the catheter body is not yet compressed.
FIG. 14 is a drawing which shows the modified example of the catheter body, and shows a state that the shape of the catheter body is compressed.
FIG. 15 is a drawing which shows a modified example of the catheter body, and shows a state that the shape of the catheter body is not yet compressed.
FIG. 16 is a perspective view which describes characteristics of the shape of the balloon of the urinary catheter according to a preferred embodiment of the present invention.
FIG. 17 is a perspective view which describes the characteristics of the shape of the balloon of the urinary catheter according to a preferred embodiment of the present invention.
FIG. 18 is a sectional view which describes the characteristics of the balloon of the urinary catheter according to a preferred embodiment of the present invention.
FIG. 19 is a drawing which describes the effects of the urinary catheter according to a preferred embodiment of the present invention.

### Description of Embodiments

Hereinafter, modes of executing the present invention will be described in detail with reference to attached drawings.

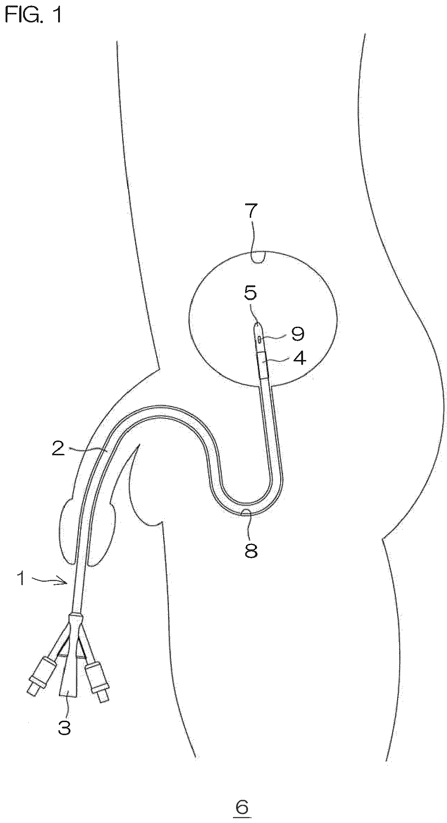

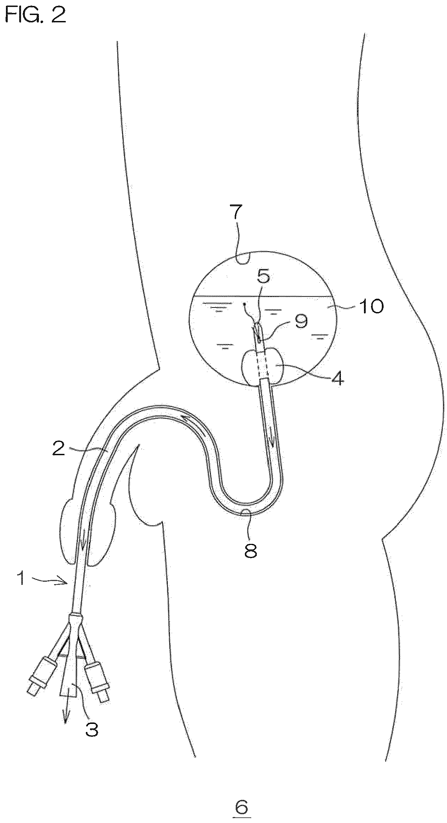

FIG. 1 is a drawing which shows a state that a urinary catheter 1 according to a preferred embodiment of the present invention is used, and shows a state that a balloon 4 has not yet expanded inside a bladder. FIG. 2 is a drawing which shows a state that the urinary catheter 1 according to a preferred embodiment of the present invention is used, and shows an expanded state of the balloon 4 inside a bladder.

The urinary catheter 1 is a device for assisting urethral catheterization of a patient who has difficulty in urination and provided with a catheter body 2, an operating portion 3 and a balloon 4. As shown in FIG. 1, in a state that the balloon 4 has not yet expanded, the catheter is used by insertion of the catheter body 2 into the urethra 8 of a human body 6 until a tip end portion 5 thereof reaches the bladder 7 of the human body 6. After the insertion, the balloon 4 is expanded and fixed, thereby preventing falling-off of the catheter body 2. Then, the catheter body 2 is placed inside the bladder 7.

Then, urine 10 stored in the bladder 7 passes through the inside of the catheter body 2 from a first urine draining port 9 formed at a tip end portion 5 of the catheter body 2 and is drained from the operating portion 3.

Next, a more detailed description will be given of a configuration of the urinary catheter 1 according to a preferred embodiment of the present invention.

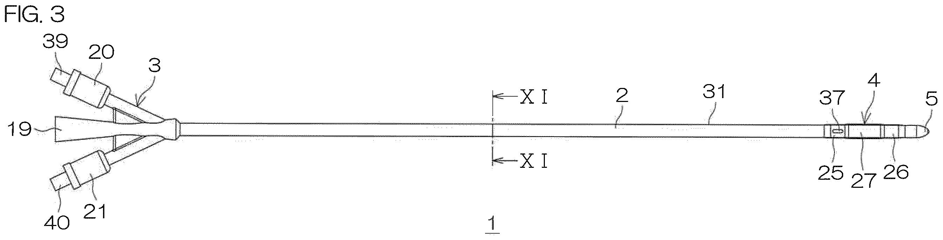

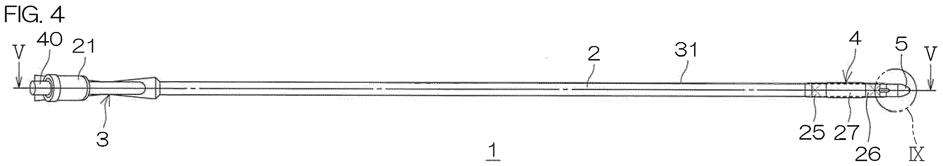

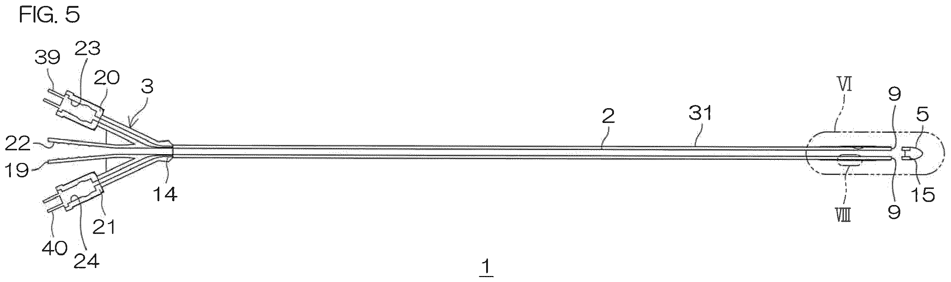

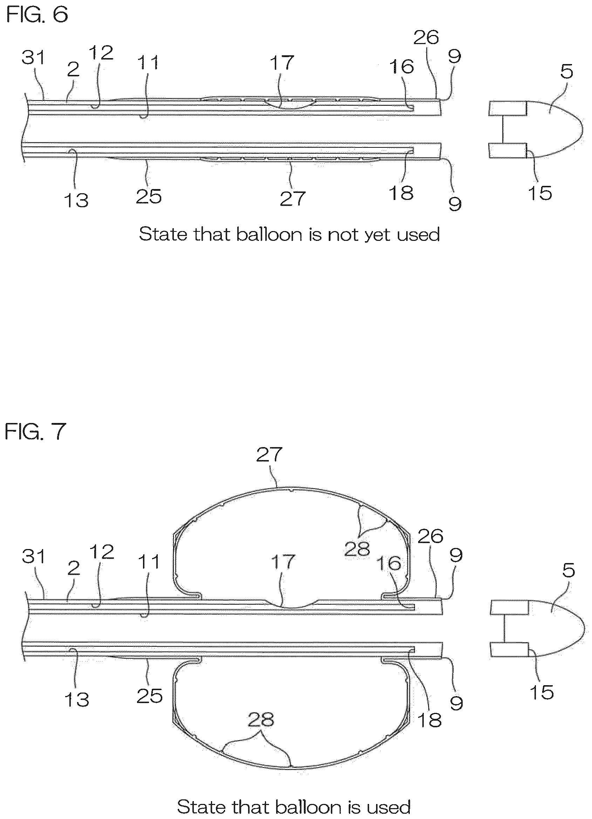

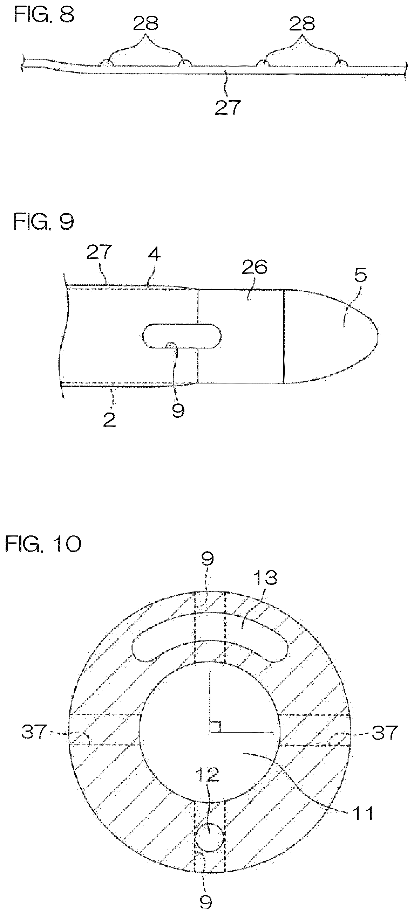

FIG. 3 is a plan view of the urinary catheter 1 according to a preferred embodiment of the present invention. FIG. 4 is a side view of the urinary catheter 1 according to a preferred embodiment of the present invention. FIG. 5 is a sectional view taken along section line V-V in FIG. 4. FIG. 6 is an enlarged view of a portion surrounded by alternate long and two short dashed line VI in FIG. 5, and a drawing which shows a state that the balloon 4 is not yet used. FIG. 7 is an enlarged view of the portion surrounded by alternate long and two short dashed line VI in FIG. 5, and a drawing which shows a state that the balloon 4 is used. FIG. 8 is an enlarged view of a portion surrounded by alternate long and two short dashed line VIII in FIG. 5 and the catheter body 2 is not shown. FIG. 9 is an enlarged view of a portion surrounded by alternate long and two short dashed line IX in FIG. 4.

First, as shown in FIG. 3 to FIG. 5, the urinary catheter 1 is provided with the catheter body 2, the operating portion 3 and the balloon 4.

The catheter body 2 is composed of a flexible tube, and the hard tip end portion 5 is attached to the tip end thereof. For example, the catheter body 2 is inserted into the cap-shaped tip end portion 5 to give a male and female structure, and they are fixed to each other by subjecting a mating surface of the male and female structure to welding, adhesion or other treatment.

The catheter body 2 and the tip end portion 5 may be configured by giving surface treatment to a base member, for example, a rubber latex substrate material such as natural rubber latex and synthetic rubber latex, a silicone substrate material or a thermoplastic elastomer. Surface treatment includes, for example, hydrophilic coating which imparts lubricity to a base member, urethane coating or fluorine coating which imparts smoothness to a base member, and silver coating which imparts antibacterial actions to a base member. Two or more types of the surface treatment may be used in combination.

As shown in FIG. 6 and FIG. 7, a urine draining lumen 11, a liquid introducing lumen 12 and a shape changing lumen 13 are formed on the catheter body 2 so as to extend along a longitudinal direction of the catheter body 2.

The urine draining lumen 11 is a passage for leading urine 10 inside the bladder 7 of a patient to the outside of the body of the patient and penetrates from a base portion 14 (refer to FIG. 5) of the catheter body 2 which is one end thereof in the longitudinal direction to a tip end portion 15 of the other end thereof, and it is formed substantially at the center of the catheter body 2. The urine draining lumen 11 communicates with the first urine draining port 9 formed at the tubular tip end portion 5 and urine will enter into the urine draining lumen 11 by way of the first urine draining port 9. The first urine draining port 9 is formed so as to give a slightly vertically long elliptic shape in the longitudinal direction of the catheter body 2 (refer to FIG. 9) and the pair of them may be provided, for example, so as to face each other in a radial direction of the catheter body 2 (refer to FIG. 6 and FIG. 7).

As shown in FIG. 3, there is also formed, on the catheter body 2, a second urine draining port 37 which communicates with the urine draining lumen 11 on the opposite side of the first urine draining port 9 in relation to the balloon 4 in the longitudinal direction of the catheter body 2. More specifically, the second urine draining port 37 is formed so as to penetrate through a first end portion 25 of the balloon 4 which will be described later.

As with the first urine draining port 9, the second urine draining port 37 is formed so as to give a slightly vertically-long elliptic shape in the longitudinal direction of the catheter body 2, and the pair of them may be provided, for example, so as to face each other in the radial direction of the catheter body 2 . Further, as shown in FIG. 10, the second urine draining port 37 is disposed at a position which avoids the liquid introducing lumen 12 and the shape changing lumen 13 in a cross sectional view of the catheter body 2 in a radial direction, that is, at a position at which the first urine draining port 9 is turned by 90 degrees, in this preferred embodiment. Thereby, interference of the second urine draining port 37 with the liquid introducing lumen 12 and the shape changing lumen 13 is prevented.

The liquid introducing lumen 12 is a passage for introducing an expansion liquid into the balloon 4 and formed so as to extend along the urine draining lumen 11. The liquid introducing lumen 12 is formed up to the middle part of the catheter body 2 in the longitudinal direction from the base portion 14, with the base portion 14 of the catheter body 2 given as an open end, and the terminal portion thereof is given as a dead end portion 16. The dead end portion 16 is disposed between the first urine draining port 9 and a balloon circulating port 17 (to be described later). A balloon circulating port 17 which communicates with the liquid introducing lumen 12 is also formed on a circumferential surface 31 of the catheter body 2.

The shape changing lumen 13 is a passage for changing the shape of the catheter body 2 so that the urinary catheter 1 can be easily inserted or removed, and formed so as to extend along the urine draining lumen 11. The shape changing lumen 13 is formed up to the middle part of the catheter body 2 in the longitudinal direction from the base portion 14, with the base portion 14 of the catheter body 2 given as an open end, and the terminal portion thereof is given as a dead end portion 18. The dead end portion 18 is disposed between the first urine draining port 9 and the balloon circulating port 17 and at a position equal in length to the dead end portion 16 of the liquid introducing lumen 12 in the longitudinal direction of the catheter body 2. Further, unlike the liquid introducing lumen 12, the shape changing lumen 13 does not circulate to the outside through an opening, etc., such as the balloon circulating port 17 but circulates through the outside only at an open end thereof.

The operating portion 3 is a portion which is handled by medical personnel such as a doctor, a nurse, etc., and integrally provided with a urine draining port 19, a liquid introducing port 20 and a shape changing port 21. As shown in FIG. 3 to FIG. 5, the operating portion 3 may be formed so as to assume a three-branched shape in which the liquid introducing port 20 and the shape changing port 21 are branched to each other in a symmetrical manner from a circumferential surface of the funnel-shaped urine draining port 19 extending on an extended line of the catheter body 2. The operating portion 3 may be made of the same material as that of the catheter body 2, for example, and fixed to the catheter body 2 by insert molding into the catheter body 2. It is noted that the operating portion 3 may be made of a material different from the catheter body 2 and, in this case, it may be fixed to the catheter body 2 by insert molding, welding, adhesion, etc.

Further, as shown in FIG. 5, a urine draining passage 22, a liquid introducing passage 23 and a shape changing passage 24 are formed respectively at the urine draining port 19, the liquid introducing port 20 and the shape changing port 21 in a mutually independent manner. The urine draining passage 22 communicates with the urine draining lumen 11, the liquid introducing passage 23 communicates with the liquid introducing lumen 12, and the shape changing passage 24 communicates with the shape changing lumen 13.

A container such as a urine collection bag for storing drained urine is connected to the urine draining port 19. Further, for example, valves 39, 40 for connecting a syringe are provided at the tip ends of the liquid introducing port 20 and the shape changing port 21. Medical personnel such as a doctor or a nurse connect a syringe at which a balloon expanding liquid is filled to the valve 39 and pushes a plunger of the syringe, by which the liquid can be injected into the balloon 4 by way of the liquid introducing passage 23 and the liquid introducing lumen 12. Further, medical personnel such as a doctor or a nurse connect an empty syringe to the valve 40 and pull the plunger of the syringe, by which air inside the shape changing passage 24 and the shape changing lumen 13 can be housed in the syringe to make the inside of the shape changing lumen 13 negative in pressure.

The balloon 4 is provided with a first end portion 25 fixed so as to surround the catheter body 2 and a second end portion 26 closer to the side of the tip end portion 15 than the first end portion 25 and also provided with an expansion portion 27 composed of a membrane which is installed between the first end portion 25 and the second end portion 26 to spherically expand inside the bladder 7 of a patient. The balloon 4 is connected to the balloon circulating port 17 inside the expansion portion 27. Further, the first end portion 25 and the second end portion 26 of the balloon 4 may be fixed to the catheter body 2 by, for example, welding, adhesion, etc. Still further, materials of the balloon 4 include, for example, rubber latex, silicone, and thermoplastic elastomer.

As shown in FIG. 8, a plurality of ribs 28 may also be installed on an inner surface of the expansion portion 27 along a circumferential direction of the catheter body 2. The plurality of ribs 28 are formed so as to surround the catheter body 2 individually in an annular shape and disposed along the longitudinal direction of the catheter body 2 at intervals from each other.

Then, medical personnel such as a doctor or a nurse inject an expanding liquid such as sterile distilled water into the liquid introducing passage 23 using a syringe, by which the expanding liquid enters into the balloon 4 by way of the liquid introducing lumen 12 and the balloon circulating port 17 and, as shown in FIG. 7, the balloon 4 expands spherically.

Next, a more detailed description will be given of a configuration of the catheter body 2.

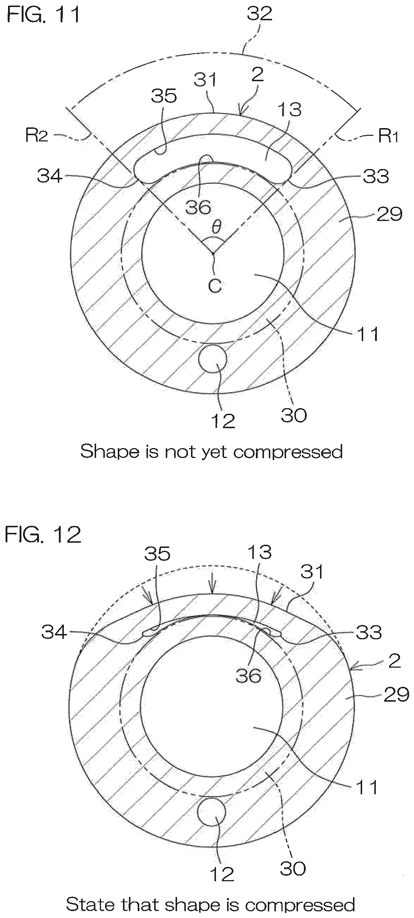

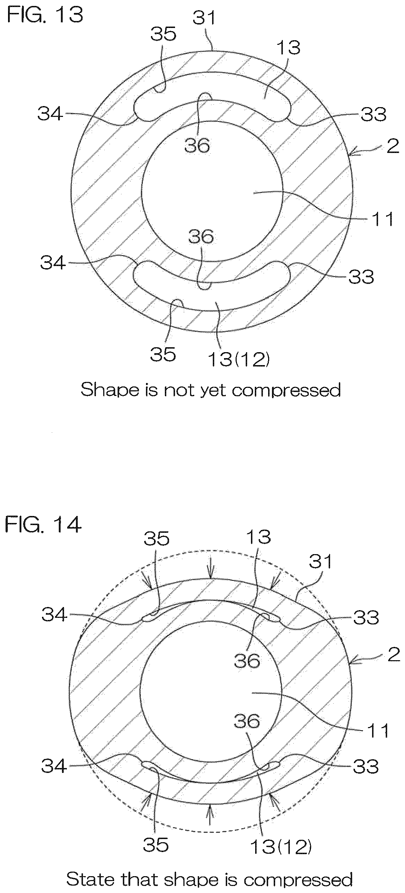

FIG. 11 is a sectional view taken along section line XI-XI in FIG. 3, and shows a state that the shape of the catheter body 2 is not yet compressed. FIG. 12 is a sectional view taken along section line XI-XI in FIG. 3, and shows a state that the shape of the catheter body 2 is compressed. FIG. 13 is a drawing which shows a modified example of the catheter body 2, and shows a state that the shape of the catheter body 2 is not yet compressed. FIG. 14 is a drawing which shows the modified example of the catheter body 2, and shows a state that the shape of the catheter body 2 is compressed.

First, with reference to FIG. 11 and FIG. 12, as described previously, the catheter body 2 may be in its entirety configured with a flexible soft member such as a rubber latex substrate material or a silicone substrate material. It may have, for example, a two-layer structure composed of an outer layer 29 in contact with an inner wall (mucous membrane portion) of the urethra 8 and an inner layer 30 formed inside the outer layer 29 to form an inner wall of the urine draining lumen 11. The inner layer 30 is preferably made of a member which is harder than the outer layer 29.

That is, it is preferable that the catheter body 2 has such a configuration that a tube-shaped core material composed of the inner layer 30 relatively high in rigidity (stiffness) is coated with the outer layer 29 which is softer and more easily changed in shape than the core material. Thereby, medical personnel such as a doctor or a nurse who insert the urinary catheter 1 find such an advantage that the urinary catheter 1 can be inserted easily due to rigidity of the inner layer 30 despite a certain resistance on insertion, while a patient into whom the urinary catheter 1 is inserted finds such an advantage that pain is relieved because the portion in contact with the inner wall of the urethra 8 is soft.

The outer layer 29 may have a configuration that surface treatment is given, for example, to the previously described rubber latex substrate material such as a natural rubber latex and a synthetic rubber latex or the base member such as a silicone substrate material. On the other hand, the inner layer 30 may be made of hard silicone or thermoplastic elastomer, for example. Further, as with the outer layer 29, the previously described surface treatment may be given to the inner layer 30.

Further, the catheter body 2 is constant in outer diameter from the base portion 14 to the tip end portion 15 (refer to FIG. 5), having an outer diameter of 2 mm to 10 mm, for example.

Next, a description will be given of the position and the shape of each of the urine draining lumen 11, the liquid introducing lumen 12 and the shape changing lumen 13 of the catheter body 2.

The urine draining lumen 11 is formed so as to give a circular shape at the center of the catheter body 2 in a cross sectional view of the catheter body 2 in a radial direction. The urine draining lumen 11 may be, for example, about 2 mm to 5 mm in inner diameter.

The liquid introducing lumen 12 is formed in a circular shape with a diameter smaller than that of the urine draining lumen 11 around the urine draining lumen 11. The liquid introducing lumen 12 may be, for example, about 0.1 mm to 0.8 mm in inner diameter.

The shape changing lumen 13 is disposed so as to face the liquid introducing lumen 12 in relation to the urine draining lumen 11 around the urine draining lumen 11. In this preferred embodiment, the shape changing lumen 13 is formed in a flat shape so as to curve along the circumferential surface 31 of the catheter body 2. More specifically, the shape changing lumen 13 is formed in a flat shape having one end 33 and the other end 34 thereof on radius line segments R₁, R₂ of a fan-shaped region 32 at which a central angle θ spreading from the center C of the urine draining lumen 11 is 90 degrees or less in a cross sectional view of the catheter body 2 in a radial direction.

Due to formation of the above-described shape changing lumen 13, medical personnel such as a doctor or a nurse pull air inside the shape changing lumen 13 using a syringe to make the inside thereof negative in pressure, by which an outer side surface 35 of the shape changing lumen 13 can be firmly attached to an inner side surface 36 thereof. Thereby, the catheter body 2 can be partially compressed to decrease an outer diameter of the catheter body 2. As a result, it is possible to relieve pain felt by a patient upon insertion of the urinary catheter 1.

Where the catheter body 2 has a two-layer structure composed of the inner layer 30 and the outer layer 29, the shape changing lumen 13 is preferably formed on the outer layer 29 which is relatively soft. It is, thereby, possible to easily compress the catheter body 2.

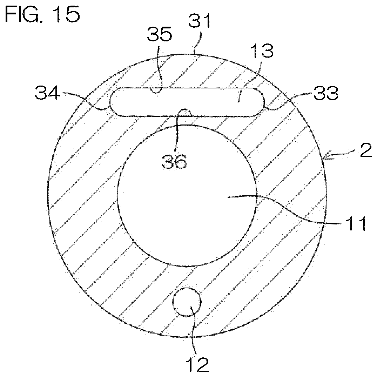

Further, as shown in FIG. 11 and FIG. 12, the shape changing lumen 13 may be formed singularly on the catheter body 2. However, as shown in FIG. 13 and FIG. 14, the plurality of shape changing lumens 13 may be formed. In this case, one of the shape changing lumens 13 (second shape changing lumen) may be, as described previously, such that the tip end portion side thereof is blocked by the dead end portion 18, while the other of the shape changing lumens 13 (first shape changing lumen) may be formed so as to communicate with the balloon 4 by way of the balloon circulating port 17 and also used as the liquid introducing lumen 12. Even when used as the liquid introducing lumen 12, the other of the shape changing lumens 13 assumes a mode that circulates with the outside only at the open end of the liquid introducing lumen 12 due to the fact that the balloon 4 is blocked, by which the inside of the lumen can be made negative in pressure. The plurality of shape changing lumens 13 may be disposed so as to face each other in relation to the urine draining lumen 11. Where the plurality of shape changing lumens 13 are formed, it is possible to compress the catheter body 2 so as to be smaller in diameter. Further, as shown in FIG. 15, the shape changing lumen 13 is formed in a flat shape along the circumferential surface 31 of the catheter body 2, and it may not necessarily curve along the circumferential surface 31.

Next, a more detailed description will be given of a configuration of the balloon 4.

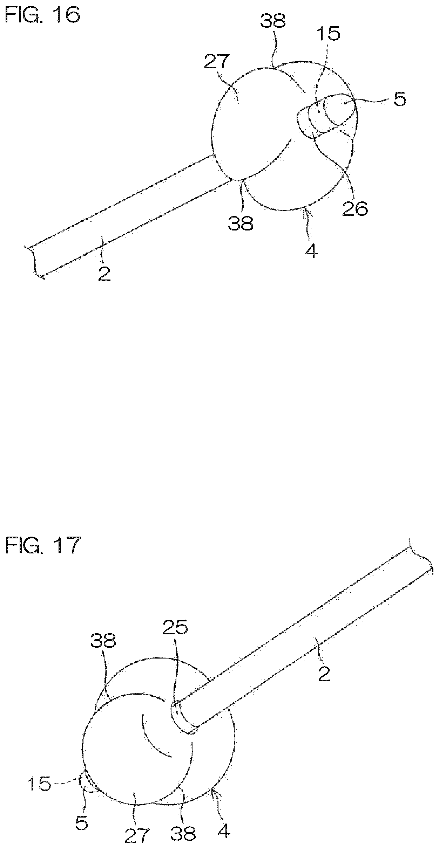

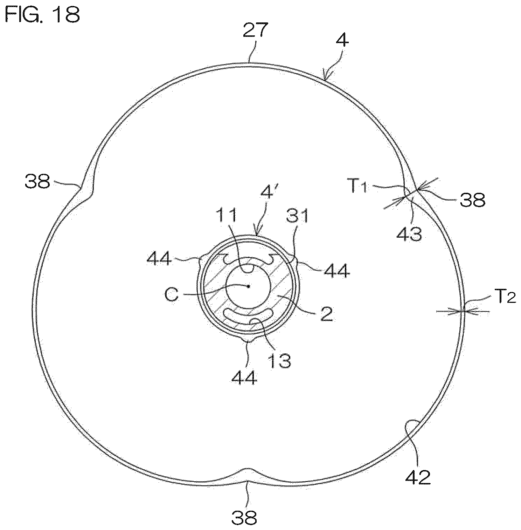

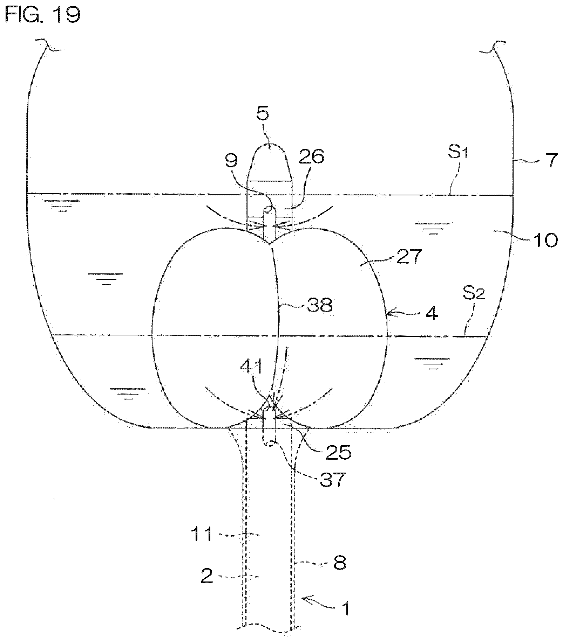

FIG. 16 and FIG. 17 are each a perspective view which describes characteristics of the shape of the balloon 4 of the urinary catheter 1, and shows a state that the balloon 4 has expanded. FIG. 16 is a drawing that the balloon 4 is viewed on the side of the tip end portion 15 of the catheter body 2, and FIG. 17 is a drawing that the balloon 4 is viewed from the opposite side of the tip end portion 15 of the catheter body 2. FIG. 18 is a sectional view which describes the characteristics of the shape of the balloon of the urinary catheter 1. FIG. 19 is a drawing which describes the effects of the urinary catheter 1.

In this preferred embodiment, as shown in FIG. 16 and FIG. 17, the balloon 4 has a linear recessed portion 38 (recessed groove) extending from the first end portion 25 toward the second end portion 26 along the circumferential surface thereof. The recessed portion 38 is formed such that the first end portion 25 is given as a starting end and the second end portion 26 is given as a terminal end. Thereby, in a state that the expansion portion 27 of the balloon 4 which has expanded, for example, as shown in FIG. 19, is brought into contact with an inner wall of the bladder 7 (for example, the bladder neck), a certain space 41 is to be formed between the expansion portion 27 and the inner wall of the bladder 7. Then, the second urine draining port 37 is formed at a predetermined position of the first end portion 25 of the balloon 4 so as to be exposed to the space 41.

As shown in FIG. 18, the recessed portions 38 are formed annularly along the circumferential surface 31 of the catheter body 2 at equal intervals from each other. For example, in this preferred embodiment, a total of three recessed portions 38 are formed around the center C thereof (the center of the urine draining lumen 11) in a cross sectional view of the catheter body 2 in a radial direction, at intervals of 120 degrees from each other. It is noted that the recessed portions 38 are not limited to three but may be four or more.

Further, as shown in FIG. 18, the inner wall of the balloon 4 in an expanded state is formed as a raised portion 43 in which a site where the recessed portion 38 has been formed protrudes inwardly with respect to another planar region 42 in a cross sectional view of the catheter body 2 in a radial direction. On the other hand, "a balloon 4'" in FIG. 18 is in a state that the balloon 4 has not yet expanded, and in this state, a raised portion 44 is formed at a position corresponding to the recessed portion 38 on an outer circumferential surface of the balloon 4' . The balloon 4 is formed so that the membrane thickness T₁ (the height of the raised portion 43 or 44) of a site where the recessed portion 38 has been formed will be thicker than the membrane thickness T₂ of the other site (planar region 42) . For example, the membrane thickness T₁ is 0.3 mm to 1.5 mm and the membrane thickness T₂ is 0.05 mm to 0.7 mm. That is, at a time when the balloon 4 has not yet expanded, the raised portion 44 is formed, by which at a time of expansion of the balloon 4, there develops a difference in degree of expansion between the raised portion 44 and the other portion and after the expansion, the recessed portion 38 is formed at a site where the raised portion 44 is formed.

As described so far, with reference to FIG. 19, in a state that the expansion portion 27 of the expanded balloon 4 is allowed to be in contact with the inner wall of the bladder 7 (for example, the bladder neck), the certain space 41 is formed between the expansion portion 27 and the inner wall of the bladder 7. Then, the plurality of recessed portions 38 are formed in the balloon 4 so as to enter into the space 41.

Where a liquid surface position of urine 10 in the bladder 7 is at a position higher than the first urine draining port 9 as in the case of a liquid surface S₁ in FIG. 19, the urine 10 can be drained by way of the first urine draining port 9. On the other hand, as in the case of a liquid surface S₂ in FIG. 19, where the liquid surface of the urine 10 is at a position lower than the first urine draining port 9, it is difficult to drain the urine by way of the first urine draining port 9. Even in this case, use of the urinary catheter 1 allows the urine 10 present at a position lower than the first urine draining port 9 to flow downward along the recessed portion 38 and guides the urine down to the space 41, thus making it possible to drain the urine from the second urine draining port 37 which is exposed to the space 41. As a result, it is possible to easily drain the urine 10 stored from the inner wall of the bladder 7 up to the top of the balloon 4, while the balloon 4 maintains a function for preventing falling-off of the catheter.

A description has been so far given of the preferred embodiments of the present invention. However, the present invention can be carried out in other modes.

For example, in the previously described preferred embodiment, a description has been given of only an example of the urinary catheter 1 on which the shape changing lumen 13 is formed. However, from a viewpoint of the present invention that "while the balloon portion maintains a function for preventing falling-off of the catheter, urine stored from the inner wall of the bladder up to the top of the balloon portion is easily drained," the shape changing lumen 13 may not be formed.

In addition, the design of the present invention may be modified in various ways without departing from the scope described in the claims.

The present application corresponds to Japanese Patent Application No. 2017-172174 filed in the Japan Patent Office on September 7, 2017 and the entire disclosure of this application is incorporated herein by reference.

### Reference Signs List

- 1:: Urinary catheter
- 2:: Catheter body
- 3:: Operating portion
- 4:: Balloon
- 5:: Tip end portion
- 6:: Human body
- 7:: Bladder
- 8:: Urethra
- 9:: First urine draining port
- 10:: Urine
- 11:: Urine draining lumen
- 12:: Liquid introducing lumen
- 13:: Shape changing lumen
- 14:: Base portion (of catheter body)
- 15:: Tip end portion (of catheter body)
- 16:: Dead end portion
- 17:: Balloon circulating port
- 18:: Dead end portion
- 19:: Urine draining port
- 20:: Liquid introducing port
- 21:: Shape changing port
- 22:: Urine draining passage
- 23:: Liquid introducing passage
- 24:: Shape changing passage
- 25:: First end portion (of balloon)
- 26:: Second end portion (of balloon)
- 27:: Expansion portion (of balloon)
- 28:: Rib
- 29:: Outer layer
- 30:: Inner layer
- 31:: Circumferential surface
- 32:: Fan-shaped region
- 33:: One end (of shape changing lumen)
- 34:: Other end (of shape changing lumen)
- 35:: Outer side surface (of shape changing lumen)
- 36:: Inner side surface (of shape changing lumen)
- 37:: Second urine draining port
- 38:: Recessed portion
- 39:: Valve
- 40:: Valve
- 41:: Space
- 42:: Planar region
- 43:: Raised portion
- 44:: Raised portion

## Claims

1. A urinary catheter comprising:
a catheter body composed of a flexible tube having a tip end portion which is placed inside the bladder of a patient and a base portion on the opposite side thereof;
a urine draining lumen which is formed inside the catheter body to lead urine inside the bladder of the patient to the outside of the body of the patient;
a balloon portion which is provided with a first end portion fixed so as to surround the catheter body at the tip end portion side of the catheter body and a second end portion closer to the tip end portion side than the first end portion and which is composed of a membrane in which a portion between the first end portion and the second end portion expands spherically inside the bladder of the patient, and a balloon portion which has a linear recessed portion extending from the first end portion toward the second end portion along a circumferential surface thereof in an expanded state;
a liquid introducing lumen which is formed by extending along the urine draining lumen inside the catheter body so as to communicate with the balloon portion, thereby introducing an expansion liquid into the balloon portion;
a first urine draining port which is formed in the catheter body so as to be closer to the tip end portion side than the balloon portion to communicate with the urine draining lumen; and
a second urine draining port which is formed in the vicinity of the first end portion of the balloon portion in the catheter body to communicate with the urine draining lumen.

2. The urinary catheter according to Claim 1, wherein
the balloon portion is such that a site where the recessed portion is formed is thicker in membrane thickness than a site other than that in a cross sectional view of the catheter body in a radial direction.

3. The urinary catheter according to Claim 2, wherein
in the balloon portion, the site where the recessed portion is formed is 0.3 mm to 1.5 mm in membrane thickness and a site other than the site where the recessed portion is formed is 0.05 mm to 0.7 mm in membrane thickness.

4. The urinary catheter according to any one of Claim 1 to Claim 3, wherein
the plurality of linear recessed portions are formed at intervals from each other in a cross sectional view of the catheter body in a radial direction.

5. The urinary catheter according to any one of Claim 1 to Claim 4, wherein
an inner wall of the balloon portion includes a raised portion at which a site where the recessed portion has been formed selectively protrudes in a cross sectional view of the catheter body in a radial direction in an expanded state.
